# EUROPEAN PATENT APPLICATION

(11) **EP 4 475 133 A1**
(43) Date of publication of application: **11.12.2024**
(21) Application number: 24179653.1
(22) Date of filing: 03.06.2024
(51) Int. Cl.: G16H 10/60, G16H 40/67, H04L 9/40

(54) **COMPUTER IMPLEMENTED METHOD FOR SECURE HEALTHCARE DATA TRANSFER BETWEEN A HOST DEVICE AND AN ALGORITHM SERVICE**

(30) Priority: 05.06.2023 EP 23177234
(71) Applicant: Roche Diagnostics International AG, 6343 Rotkreuz (CH)
(72) Inventor: GOESSI, Cyrill Martin, Lucerne (CH)
(74) Representative: Mewburn Ellis LLP

(57) **Abstract**

A computer-implemented method of transferring healthcare data between a host device and an algorithm service, the host device residing within a first computer-network and the algorithm service residing within a second computer-network different to the first computer-network. The method including steps of: (a) establishing, between the host device and the algorithm service, a secure channel of communication; (b) transmitting, from the host device to the algorithm service via the secure channel of communication: an identification of an algorithm to be applied to healthcare data held by the host device, and the healthcare data to which the identified algorithm is to be applied; and (c) receiving, at the algorithm service, the identification of the algorithm and the healthcare data; (d) applying the identified algorithm to the healthcare data to arrive at a result; and (e) transmitting, from the algorithm service to the host via the secure channel of communication, the result of applying the identified algorithm to the healthcare data.

## Description

### TECHNICAL FIELD

The present disclosure relates to a computer-implemented method, a host device, an algorithm service, and an algorithm service.

### BACKGROUND

When developing medical algorithm devices, this is typically done in the form of local host only applications for process efficiency. They are also typically based on an architectural pattern complete enough to implement the medical algorithm device (and so provide a result), but not implementing any security measures to protect the sensitive personal data handled and without implementing any service functionalities such as audit trails.

This minimally complete architecture pattern allows for the efficient development of medical algorithm devices as all instances reusing the same architectural pattern leads to very similar local host only systems. This allows for an efficient and streamlined thread model, risk assessment, and penetration test, across all of the medical algorithm devices developed using the same pattern.

However, on the other hand, it is desirable to integrate medical algorithm devices into a networked service environment providing healthcare providers remote access to a cloud-based catalogue of medical algorithm devices which can be remotely executed in the context of a medical treatment of a patient, as well as providing service functionalities such as the notion of users, access permissions, and audit trails.

The inventor's have recognised a problem then: how can medical algorithm devices, that were developed, threat modelled, risk assessed, and penetration tested as local host only applications and don't implement nay security measures to protect sensitive personal data handled, be securely integrated into a networked service environment. Further, they have recognised that the network service environment should be able to state that the security provided to the sensitive personal data exchanged is equivalent to the security provided by the local host environment that the medical algorithm devices were developed for, and under which the medical algorithm devices were thread modelled, risk assessed, and penetration tested.

The present disclosure was arrived at in light of the above considerations.

### SUMMARY

In a first aspect, embodiments of the invention provide a computer-implemented method of transferring healthcare data between a host device and an algorithm service, the host device residing within a first computer-network and the algorithm service residing within a second computer-network different to the first computer-network, the method including steps of:
(a) establishing, between the host device and the algorithm service, a secure channel of communication;
(b) transmitting, from the host device to the algorithm service via the secure channel of communication:
   an identification of an algorithm to be applied to the healthcare data held by the host device, and
   the healthcare data to which the identified algorithm is to be applied; and
(c) receiving, at the algorithm service, the identification of the algorithm and the healthcare data;
(d) applying the identified algorithm to the healthcare data to arrive at a result; and
(e) transmitting, from the algorithm service to the host via the secure channel of communication, the result of applying the identified algorithm to the healthcare data.

Such a method allows for medical algorithms to be developed as local host only applications with a minimally complete architecture pattern, and without implementing any security measures to protect sensitive healthcare data. This allows for efficient development of the medical algorithm devices, which are secure in their handling of sensitive healthcare data.

The first computer-network and second computer-network may each include, or be separated by, one or more firewalls. The or each firewall may control the ingress and egress of data in the respective computer-networks. The first computer-network may have a security configuration which is different to, and/or is controlled by a different entity than, the second computer-network. For example, the first computer-network may define or reside within a first domain of control and the second computer-network may define or reside within a second domain of control.

The host device may be a computer, smartphone, tablet, or other personal computing device. The algorithm service may be a computer, server, collection of computers, collection of servers, cloud computing environment, and/or virtualised machine implemented on any of the same. The algorithm service may be considered software, a suite of software running on a computing device, and/or implemented as one or more containers in a cloud computing environment. It may be that the host device does not apply the algorithm to the healthcare data. That is, the algorithm identified in step (b) may only be applied by the algorithm service (which may be a different entity to the host device). The algorithm service may comprise one or more instances, for example one or more cloud instances, so as to apply the algorithm in parallel.

The algorithm(s) provided by the algorithm service may comprise step-by-step procedures or sets of instructions that are designed to solve a specific problem or perform a particular task. They are a finite sequence of well-defined, unambiguous instructions that are executed in a specific order to achieve a desired result. For example, an algorithm provided by the algorithm service may receive, as healthcare data, patient data relating to cardiovascular health. The algorithm may perform one or more steps on or using the healthcare data and provide as a result an indication as to the risk of a myocardial infarction.

The identification of the algorithm may be, for example, a label which identifies a specific algorithm or class of algorithms to be applied to the healthcare data. The host device may retain a list of algorithms the algorithm service provides, the list may be provided by the algorithm service to the host device.

The transmission of the identification of the algorithm and the healthcare data may be performed simultaneously, or sequentially, in either order.

The healthcare data may include, for example, physiological data relating to a patient, analytical test results relating to the patient, metadata relating to the patient (e.g. 'smoker'), and/or metadata relating to one or more tests performed on the patient or samples derived from the patient. The healthcare data may be stored locally on the host device, or may be retrieved on instruction from a user of the host device. When the retrieval is instructed, the healthcare data may be stored, for example on a laboratory information system (LIS) or healthcare information system (HIS). The LIS or HIS may contain, or be linked to, the database referred to below.

The method may further include transmitting, from the algorithm service to the host device, GUI instructions which, when executed by the host device, cause the generation of a graphical user interface, the GUI instructions selected by the algorithm service based on the algorithm identified by the host device. The GUI instructions may comprise a HTML document for rendering by a browser connected to or hosted on the host device. In some examples, the algorithm service initiates an instance of the algorithm identified by the host device, and the initiated algorithm provides the GUI instructions to the algorithm service for onward transmission to the host device. The algorithm service may add some additional instructions to the GUI instructions after receipt from the algorithm and before transmission to the host device, such as a header.

The method may further include establishing, between the host device and a database, a second secure channel of communication, and transmitting from the host device to the database through the second secure channel, an identification of a patient and one or more data field identifiers, the database returning a patient specific value for the or each data field identifier, wherein the patient specific value(s) are included or form the healthcare data transmitted from the host device to the algorithm service. The second secure channel of communication may be established using TLS. Establishing the second secure channel of communication may include the host device authenticating the identity of the database. Establishing the second secure channel of communication may include the host device and database each encrypting messages to be sent between each other. Further, the secure channel of communication between the host device and the algorithm service may be established using TLS.

The method may further include a step, between steps (c) and (d), of transmitting, from the algorithm service to the host device, confirmatory instructions indicating which algorithm is to be applied to what healthcare data. Step (b) may further include transmitting a pre-population request that the algorithm service return a pre-populated GUI, pre-populated with the healthcare data to which the identified algorithm is to be applied, the algorithm service transmitting the confirmatory instructions in response to receiving the pre-population request. The confirmatory instructions may comprise confirmatory GUI instructions which cause the generation of a pre-populated graphical user interface illustrating the healthcare data provided and the algorithm to be applied. The method may further comprise receiving, at the host device, one or more change requests relating to the pre-populated graphical user interface, and transmitting these change requests to the algorithm service. For example, the GUI may include one or more input fields for receiving change requests. The algorithm service can then implement the one or more changes, and send back to the host device amended confirmatory GUI instructions reflecting the one or more changes. The method may further comprise receiving, at the host device, an acceptance of the pre-populated graphical user interface and transmitting this to the algorithm service. The algorithm service can then apply the algorithm as specified in the pre-populated graphical user interface.

The method may further comprise transmitting, from the host device to the algorithm service, an execution request that the algorithm service apply the identified algorithm to the healthcare data, the algorithm service performing steps (d) and (e) in response to receiving the execution request. In some examples, the execution request includes the identification of the algorithm to be applied and the healthcare data to which the identified algorithm is to be applied. In some examples, the execution request references an existing pending request in the algorithm service (the algorithm service having retained the previously transmitted identification of the algorithm and the healthcare data).

The method may further include a step of transforming the result of applying the algorithm to the healthcare data before transmitting it to the host device and/or a step of transforming the healthcare data before transmitting it to the algorithm service. For example, the healthcare data may be transformed from one numerical format to another.

The algorithm service may include an API and the host device including an edge module, the API and the edge module being in secure communication via the secure channel of communication. The algorithm service may include an algorithm module or algorithm device, which may be connected to the API via an insecure channel and/or is located within the second computer-network (or indeed may be connected to the API via a secure channel if desired). The algorithm module or device may implement a medical algorithm in that it performs computations on healthcare data to arrive at a result. The host device may include an interface module, which is connected to the edge module via an insecure channel and/or is located in the first computer-network. The interface module may be a browser or other interface through which a clinician interacts with the host device.

Establishing the secure channel of communication may include the host device authenticating the identity of the algorithm service. In some examples, establishing the secure channel of communication may also include the algorithm service authenticating the identity of the host device. Establishing the secure channel of communication may include the host device and the algorithm service each encrypting messages sent between each other. This can include, for example, the host device and algorithm service agreeing a set of secret keys to be used to encrypt and decrypt the messages sent between each other.

Establishing the secure channel of communication may include implementing a transport layer security protocol, TLS, where the host device assumes the role of a TLS client and the algorithm service assumes the role of a TLS server.

In a second aspect, embodiments of the invention provide a host device, comprising one or more processors, memory, and a network interface, the memory containing machine executable instructions which, when executed on the processor(s) cause:
(a) the network interface to establish a secure channel of communication between the host device and an algorithm service, the host device residing within a first computer-network and the algorithm service residing within a second computer-network different to the first computer-network;
(b) the host device to transmit, to the algorithm service via the secure channel of communication:
   an identification of an algorithm to be applied to healthcare data held by the host device, and
   the healthcare data to which the identified algorithm is to be applied; and
(c) the host device to receive, from the algorithm service via the secure channel of communication, the result of applying the identified algorithm to the healthcare data.

The instructions may further cause the host device to: receive, from the algorithm service, GUI instructions which, when executed by the processor(s) cause the generation of a graphical user interface. The GUI instructions may have been selected by the algorithm service based on the algorithm identified by the host device. The GUI instructions may comprise a HTML document for rendering in a browser connected to or hosted by the host device. In some examples, the algorithm service may have initiated an instance of the algorithm identified by the host device, and the initiated algorithm may have provided the GUI instructions to the algorithm service for onward transmission to the host device. The algorithm service may have added some additional instructions to the GUI instructions after receipt from the algorithm and before transmission to the host device. The steps may be prompted by, for example, a request from a third-party device such as the algorithm service (which then cause steps (a) - (c) to be performed).

The instructions may further cause the network interface to establish, between the host device and a database, a second secure channel of communication, and transmit from the host device to the database through the second secure channel, an identification of a patient and one or more data field identifiers, the host device then receiving a patient specific value for the or each data field identifier from the database, wherein the patient specific value(s) are included or form the healthcare data transmitted from the host device to the algorithm service.

The instructions may further cause the host device to receive, from the algorithm service, confirmatory instructions indicating which algorithm is to be applied to what healthcare data. Step (b) may further include transmitting a pre-population request that the algorithm service return a pre-populated GUI, pre-populated with the healthcare data to which the identified algorithm is to be applied, the algorithm service transmitting the confirmatory instructions in response to receiving the pre-population request. The confirmatory instructions may comprise confirmatory GUI instructions which cause the generation of a pre-populated graphical user interface illustrating the healthcare data provided and the algorithm to be applied. The host device may then receive one or more change requests relating to the pre-populated graphical user interface, and transmit these change requests to the algorithm service.

The instructions may further cause the host device to transmit, from the host device to the algorithm service, an execution request that the algorithm service apply the identified algorithm to the healthcare data. In some examples, the execution request includes the identification of the algorithm to be applied and the healthcare data to which the identified algorithm is to be applied. In some examples, the execution request references an existing pending request in the algorithm service (the algorithm service having retained the previously transmitted identification of the algorithm and the healthcare data).

The instructions may further cause the host device to transform the healthcare data before transmitting it to the algorithm service. For example, the healthcare data may be transformed from one numerical format to another.

The host device may include an edge module, the edge module being configurable to be in secure communication with an API of the algorithm service via the secure channel of communication. The host device may include an interface module, which is configured to connect to the edge module via an insecure channel and/or is located in the first computer-network. The interface module may be a browser or other interface through which a clinician interacts with the host device.

Establishing the secure channel of communication may include the host device authenticating the identity of the algorithm service. In some examples, establishing the secure channel of communication may also include the algorithm service authenticating the identity of the host device. Establishing the secure channel of communication may include the host device and the algorithm service each encrypting messages sent between each other. This can include, for example, the host device and algorithm service agreeing a set of secret keys to be used to encrypt and decrypt the messages sent between each other.

Establishing the secure channel of communication may include implementing a transport layer security protocol, TLS, where the host device assumes the role of a TLS client and the algorithm service assumes the role of a TLS server.

In a third aspect, embodiments of the invention provide an algorithm service, comprising one or more processors, memory, and a network interface, the memory containing machine executable instructions which, when executed on the processor(s) cause:
(a) the network interface to establish a secure channel of communication between the algorithm service and a host device, the host device residing within a first computer-network and the algorithm service residing within a second computer-network different to the first computer-network;
(b) the algorithm service to receive, from the host device via the secure channel of communication:
   an identification of an algorithm to be applied to the healthcare data held by the host device; and
   the healthcare data to which the algorithm is to be applied;
(c) the algorithm service to apply the identified algorithm to the healthcare data to arrive at a result; and
(e) the algorithm service to transmit, to the host device via the secure channel of communication, the result of applying the identified algorithm to the healthcare data.

The instructions may further cause the algorithm service to transmit, from the algorithm service to the host device, GUI instructions which, when executed by the host device, cause the generation of a graphical user interface, the GUI instructions selected by the algorithm service based on the algorithm identified by the host device. The GUI instructions may comprise a HTML document for rendering by a browser connected to or hosted on the host device. In some examples, the algorithm service initiates an instance of the algorithm identified by the host device, and the initiated algorithm provides the GUI instructions to the algorithm service for onward transmission to the host device. The algorithm service may add some additional instructions to the GUI instructions after receipt from the algorithm and before transmission to the host device. The steps may be prompted by, for example, a request from a third-party device such as the host device (which then cause steps (a) - e) to be performed).

The instructions may further cause the algorithm service, between steps (b) and (c), to transmit, from the algorithm service to the host device, confirmatory instructions indicating which algorithm is to be applied to what healthcare data. Step (b) may further include transmitting a pre-population request that the algorithm service return a pre-populated GUI, pre-populated with the healthcare data to which the identified algorithm is to be applied, the algorithm service transmitting the confirmatory instructions in response to receiving the pre-population request. The confirmatory instructions may comprise confirmatory GUI instructions which cause the generation of a pre-populated graphical user interface illustrating the healthcare data provided and the algorithm to be applied.

The instructions may further cause the algorithm service, responsive to receiving an execution request from the host device that the algorithm service apply the identified algorithm to the healthcare data, to perform steps (c) and (e). In some examples, the execution request includes the identification of the algorithm to be applied and the healthcare data to which the identified algorithm is to be applied. In some examples, the execution request references an existing pending request in the algorithm service (the algorithm service having retained the previously transmitted identification of the algorithm and the healthcare data).

The instructions may further cause the algorithm service to transform the result of applying the algorithm to the healthcare data before transmitting it to the host device.

The algorithm service may include an API, configurable to be in secure communication with an edge module of the host device via the secure channel of communication. The algorithm service may include an algorithm module or algorithm device, which is connected to the API via an insecure channel and/or is located within the second computer-network. The algorithm module or device may implement a medical algorithm in that it performs computations on healthcare data to arrive at a result.

Establishing the secure channel of communication may include the host device authenticating the identity of the algorithm service. In some examples, establishing the secure channel of communication may also include the algorithm service authenticating the identity of the host device. Establishing the secure channel of communication may include the host device and the algorithm service each encrypting messages sent between each other. This can include, for example, the host device and algorithm service agreeing a set of secret keys to be used to encrypt and decrypt the messages sent between each other.

Establishing the secure channel of communication may include implementing a transport layer security protocol, TLS, where the host device assumes the role of a TLS client and the algorithm service assumes the role of a TLS server.

In a fourth aspect, embodiments of the invention provide an edge module for a host device, configured to:
(a) establish a secure channel of communication between the edge module and an algorithm service, the edge module residing within a first computer-network and the algorithm service residing within a second computer-network different to the first computer-network;
(b) receive, from a interface module, an identification of an algorithm to be applied to healthcare data held by the host device;
(c) transmit, to the algorithm service via the secure channel of communication:
   an identification of the algorithm to be applied to the healthcare data held by the host device, and
   the healthcare data to which the identified algorithm is to be applied; and
(d) receive, from the algorithm service via the secure channel of communication, the result of applying the identified algorithm to the healthcare data.

The edge module may be configured to receive, from the algorithm service, GUI instructions which, when executed by the processor(s) cause the generation of a graphical user interface. The GUI instructions may have been selected by the algorithm service based on the algorithm identified by the interface module. The GUI instructions may comprise a HTML document for rendering in a browser connected to or hosted by the host device. In some examples, the algorithm service may have initiated an instance of the algorithm identified by the interface module, and the initiated algorithm may have provided the GUI instructions to the algorithm service for onward transmission to the host device. The algorithm service may have added some additional instructions to the GUI instructions after receipt from the algorithm and before transmission to the host device.

The edge module may be configured to establish, between the edge module and a database, a second secure channel of communication, and transmit from the edge module to the database through the second secure channel, an identification of a patient and one or more data field identifiers, the edge module then receiving a patient specific value for the or each data field identifier from the database, wherein the patient specific value(s) are included or form the healthcare data transmitted from the edge module to the algorithm service. This second secure channel of communication may have any one or any combination of the properties or features of the secure channel of communication between the host device and the algorithm service. For example, the second secure channel of communication may authenticate the identity of the database, and/or encrypt data transferred between the edge module and the database.

The edge module may be configured to receive, from the algorithm service, confirmatory instructions indicating which algorithm is to be applied to what healthcare data. Step (c) may further include transmitting a pre-population request that the algorithm service return a pre-populated GUI, pre-populated with the healthcare data to which the identified algorithm is to be applied, the algorithm service transmitting the confirmatory instructions in response to receiving the pre-population request. The confirmatory instructions may comprise confirmatory GUI instructions which cause the generation of a pre-populated graphical user interface illustrating the healthcare data provided and the algorithm to be applied. The edge module may then receive, from the interface module, one or more change requests relating to the pre-populated graphical user interface, and transmit these change requests to the algorithm service.

The edge module may be configured to transmit, to the algorithm service, an execution request that the algorithm service apply the identified algorithm to the healthcare data. In some examples, the execution request includes the identification of the algorithm to be applied and the healthcare data to which the identified algorithm is to be applied. In some examples, the execution request references an existing pending request in the algorithm service (the algorithm service having retained the previously transmitted identification of the algorithm and the healthcare data).

The edge module may be configured to transform the healthcare data before transmitting it to the algorithm service. For example, the healthcare data may be transformed from one numerical format to another.

The edge module may form a part of the host device (e.g., the host device of the second aspect), or may be a separate unit connected to the host device over a network.

Establishing the secure channel of communication may include the edge module authenticating the identity of the algorithm service. In some examples, establishing the secure channel of communication may also include the algorithm service authenticating the identity of the edge module. Establishing the secure channel of communication may include the edge module and the algorithm service each encrypting messages sent between each other. This can include, for example, the edge module and algorithm service agreeing a set of secret keys to be used to encrypt and decrypt the messages sent between each other.

Establishing the secure channel of communication may include implementing a transport layer security protocol, TLS, where the edge module assumes the role of a TLS client and the algorithm service assumes the role of a TLS server.

In a fifth aspect, embodiments of the invention provide a system comprising the host device of the second aspect and the algorithm service of the third aspect, connected by a network.

The invention includes the combination of the aspects and preferred features described except where such a combination is clearly impermissible or expressly avoided.

Further aspects of the present invention provide: a computer program comprising code which, when run on a computer, causes the computer to perform the method of the first aspect or to perform the parts of the method of the first aspect corresponding to one or both of the host device and algorithm service; a computer readable medium storing a computer program comprising code which, when run on a computer, causes the computer to perform the method of the first aspect or to perform the parts of the method of the first aspect corresponding to one or both of the host device and algorithm service; and a computer system programmed to perform the method of the first aspect or to perform the parts of the method of the first aspect corresponding to one or both of the host device and algorithm service.

### BRIEF DESCRIPTION OF THE DRAWINGS

Figure 1 is a schematic of a system;
Figure 2 is a flow chart of a method; and
Figure 3 is a network diagram.

### DETAILED DESCRIPTION

Aspects and embodiments of the present invention will now be discussed with reference to the accompanying figures. Further aspects and embodiments will be apparent to those skilled in the art.

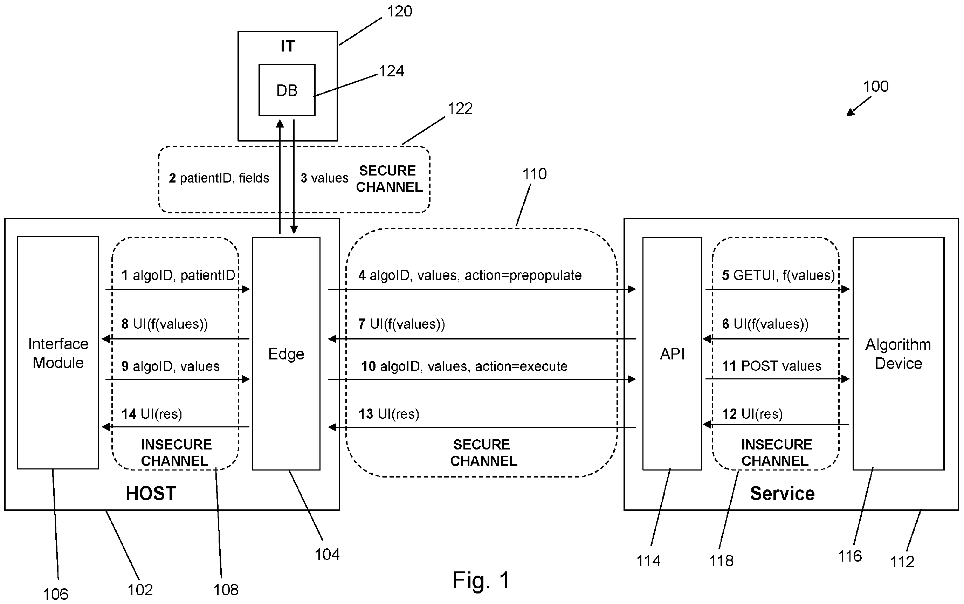

Figure 1 is a schematic of a system 100. The system includes a host device 102, and an algorithm service 112. The host device comprises an interface module (which may be referred to as a third-party agent) 106, for example a web-browser or terminal for a clinician to interact with the host device and so the edge module and on to the algorithm service. The host device also includes an edge module 104, which communicates with the interface module via a first insecure channel 108. The edge module 104 also communicates with an API 114 of the algorithm service via a first secure channel 110. The API of the algorithm service is in communication with one or more algorithm devices 116, via a second insecure channel 118. In this example, the edge module 104 is also in communication with an IT service 120 which includes a database 124. This communication is via a second secure channel 122.

During operation, the interface module provides in message **1** an identification of an algorithm to be applied to some healthcare data (algolD), and either the healthcare data itself or an identification of a patient (patientID). In the latter example, the patientID is then sent to the IT service in message **2** together with one or more data field identifiers through a second secure channel of communication 122. For example, the message **2** may specify the patient ID and also request their blood glucose concentration and weight (as two data field identifiers). The IT service can then retrieve from its database the values corresponding to those data fields for that patient (values), and return them to the edge module in message **3.**

The edge module then transmits the algolD, the values, and an action field containing a request to prepopulate a GUI, through the first secure channel 110 to the API 114. In examples where message **1** contains the healthcare data itself, messages **2** and **3** are dispensed with, and so the edge module merely forwards the contents of message **1** to the API together with the action field containing the prepopulate request.

The API, through a second insecure channel 118, sends a message **5** containing a GETUI instruction and the values received to an algorithm device 116 corresponding to the one identified by the algolD in message **4.** The values, in some examples, will be subject to a transformation some stage during the process for example by the interface module, edge module, or indeed at this stage by the API (for example by conversion from one numerical format to another). Where that is the case, the values are denoted as f(values), where f is the function transforming the values. The algorithm device then replies with a message **6** Ul(f(values)) which contains GUI instructions, for example a HTML document denoting: the values it has received and the algorithm it intends to apply to those values. This message is forwarded by the API through the first secure channel 110 to the edge module 104 as message **7,** and that message is then forwarded to the interface module 106 as message **8.**

The clinician, via the interface module 106, can then approve the application of the algorithm, or submit any change or update requests (e.g. a new algorithm to be applied, or a change in the healthcare data). Message **9** is sent to the edge module 104, in this example containing the algolD and the values again (which can either be the transformed values, the original values, or modifications of the initial or transformed values in response to a change request from the user), which is understood by the edge module to be an agreement to applying the algorithm to the data described in the UI(f(values) message. The edge module then transmits message **10,** via the first secure channel of communication 110, to the API which includes a request that the algorithm device apply the algorithm to the values. In this example, message **10** includes the algolD, the values, and an action field containing the request to execute the algorithm.

In some examples, the algorithm service 112 may store the initially transmitted values in message **4** and so when approving or requesting execution of the algorithm in message **10** the clinician may include one or more change or update requests to those values. In other examples, the algorithm service does not store the values and so on receipt of message **10** the algorithm device will be executed utilising the values and algolD received in message **10** irrespective of the values or algolD received in message **4.**

On receipt of message **10** by the API 114, it transmits a message **11** to the algorithm device 116 which requests that the algorithm be applied to the healthcare data. In this example, message **11** contains a POST request with the values encapsulated. The algorithm device 116 then applies the algorithm to the healthcare data, and provides the results in message **12.** In this example, the results are provided in the form of a HTML document giving GUI instructions to display the results (Ul(res)). This is forwarded as message **13** from the API to the edge module 104, and as message **14** from the edge module to the interface module 106.

From the perspective of a healthcare provider, the interface module still resides within the local host environment over which the healthcare provider would maintain control. Further, the algorithm device does not reside within the same network environment as the healthcare provider's interface module but instead resides in its own environment which is connected to the local host environment via a technical network and the first secure channel of communication.

Therefore, instead of directly interacting with the algorithm device 116, the interface module 106 now interacts with the edge module 104 residing within the same local host environment. The edge module is responsible for the interaction with a healthcare provider database (e.g. database 124 provided by IT service 120) for the purpose of retrieving medically relevant data relating to the patient in treatment by the healthcare provider and in which patient treatment context the overall algorithm device is triggered. The healthcare provider database DB resides in an IT environment (IT service 120) which is different to the local host environment in which the interface module and edge module reside. The IT environment and the local host environment are connected by means of a technical network, and a second secure channel of communication. The edge module interacts with the API residing in the algorithm service environment for the purpose of requesting the execution of the algorithm device, and to retrieve the algorithm result values. The algorithm service environment within which the API resides is different from the local host environment, and as discussed above is connected via a technical network and the first secure channel of communication. The API interacts with the algorithm device for the purpose of the programmatic execution of the algorithm contained within the algorithm device and retrieval of the algorithm result values.

In this example, the full calculation of the algorithm values in the context of a healthcare provider treating a patient identified by the patient identifier ID, and with the healthcare provider in need of an algorithm (e.g. a medical algorithm) identified by an algorithm identifier algolD for the purpose of informed medical decision-making, consists of two roundtrips within this networked service environment as discussed in detail above.

For the networked service environment shown in Figure 1, it is stipulated that secure channels of communication be employed across the technical network connecting the healthcare provider local host environment and the healthcare provider IT environment and across the technical network connecting the healthcare provider local host environment and the algorithm service environment.

A secure channel is a channel that simultaneously provides both confidentiality as well as authenticity to the data exchanged across the channel. If a channel provides confidentiality to the data exchanged across the channel, then the data exchanged across such a channel is only accessible to a clearly defined set of actors authorized to have access to the data. If a channel provides authenticity to the data exchanged across the channel, then the receipient of the data exchanged across such a channel can unambiguously determine the source of the data received.

For the secure channel between the healthcare provider local host environment and the healthcare provider IT environment, it is stipulated that this secure channel needs to provide confidentiality to the data exchanged in both directions. It is also stipulated that this secure channel needs to, at a minimum, provide authenticity to the data sent from the healthcare provider IT environment to the healthcare provider local host environment so that the edge module can unambiguously determine the source of the received medically relevant algorithm input values (the values in message 3) to be the database DB within the healthcare provider IT environment.

For the secure channel between the healthcare provider local host and the algorithm service environment, it is stipulated that this secure channel needs to provide confidentiality to the data exchanged in both directions. It is also stipulated that this secure channel needs to, at a minimum, provide authenticity to the data sent from the service environment to the healthcare provider local host environment so that the edge module can unambiguously determine the source of the retrieved user interface Ul populated with either the set of regulatory conforming medically relevant algorithm input values (f(values)) or populated with the algorithm result values.

In one implementation of the first and/or secure channel channels of communication, the transport layer security protocol (TLS) version 1.3 (or version 1.2) is used. If TLS is used to construct the second secure channel, then the healthcare provider local host environment would assume the role of the TLS client with the healthcare provider IT environment assuming the role of the TLS server. If TLS is used to construct the first secure channel, then the healthcare provider local host environment assumes the rule of the TLS client and the service environment assumes the role of the TLS server.

TLS as a cryptographical protocol is a protocol within which the TLS server is always authenticated as a part of the initial LTS handshake protocol (with the TLS server authentication commonly being implemented by making use of an X.509 server certificate), and within which the TLS client and TLS server arrive at a shared master secret. This can be done either by using RSA (Rivest-Shamir-Adleman) to encrypt a pre-master secret generated by the client or by using an ECDHE or DHE key exchange (elliptic curve Diffie-Hellman key exchange or Diffie-Hellman key exchange). The protocol also requires the master secret to be expanded into multiple sets of cryptographic keys (either by using PRF, a pseudorandom function, in the case of TLS 1.2 or by using HKDF in the case of TLS 1.3). The application data is exchanged between the TLS client and the TLS server, and provided with confidentiality by making use of cryptographic ciphers (e.g. AES, Advanced Encryption Standard, or ChaCha20) to encrypt the application data by using keys derived from the master secret. The application data exchanged between the TLS client and TLS server is provided with authenticity by making use of the Keyed-Hash Message Authentication Code (HMAC), in the case of TLS 1.2, or by making use of cryptographic Authenticated Encrypted with Associated Data (AEAD) ciphers in the case of TLS 1.3, where for both the HMAC or the AEAD, cipher keys derived from the master secret are used.

Alternative cryptographic protocols can be chosen as long as the alternative cryptographic protocol provides the same security guarantees for the channels constructed.

When the networked service environment implements the security measures stipulated above, the security of algorithm devices used within such a networked service environment is equivalent to the security of using the algorithm devices within a local host only environment.

The notion of security in context of using an algorithm device includes the following four requirements. First, the medically relevant algorithm input values need to be confidential to three environments: the environment of the source of the medically relevant algorithm input values, the environment of the use of the medically relevant algorithm input values, and the environment of the sink of the medically relevant algorithm input values. Second, the algorithm result values need to be confidential to two environments: the environment of the source of the algorithm result values and the environment of the sink of the algorithm result values. Third, the sink of the algorithm result values must be capable of unambiguously determining the source of the medically relevant algorithm input values used to calculate the received algorithm result values. Fourth, the sink of the algorithm result values must be capable of unambiguously determining the source of the received algorithm result values.

Under the assumption of an uncompromised local host environment, the use of an algorithm device in such an uncompromised local host environment can be seen to be secure according to the four requirements stipulated above as follows. First, the medically relevant algorithm input values are confidential to the three environments noted above. As all three environments are the same healthcare provider local host environment and assuming an uncompromised healthcare provider local host environment, the medically relevant algorithm input values are then confidential to this healthcare provider local host environment. Second, the algorithm result values are confidential to the two environments noted above. As both environments are the same healthcare provider local host environment and assuming an uncompromised healthcare provider local host environment, the algorithm result values are then confidential to this healthcare provider local host environment. Third, the sink of the algorithm result values which is the interface module within the healthcare provider local host environment can unambiguously determine the source of the medically relevant algorithm input values as the source of the medically relevant algorithm input values is the same interface module within the healthcare provider local host environment. Fourth, the sink of the algorithm result values which is the interface module residing within the healthcare provider local host environment can unambiguously determine the source of the received algorithm result values as the source of the received algorithm result values is the medical algorithm device residing within the same healthcare provider local host environment.

Implementing the stipulated security measures in a networked service environment, the use of an algorithm device in such a networked service environment implementing the stipulated security measures can be seen to be secure according to the four requirements stipulated above as follows. First, the medically relevant algorithm input values are confidential to the three environments: the environment of the source of the medically relevant algorithm input values which is the healthcare provider IT environment, the environment of the use of the medically relevant algorithm input values which is the service environment, and the environment of the sink of the medically relevant algorithm input values which is the healthcare provider local host environment. This is due to the networked service environment using a secure channel across the technical network connecting the environment of the source of the medically relevant algorithm input values with the environment of the sink of the medically relevant algorithm input values and due to the networked service environment using a secure channel across the technical network connecting environment of the sink of the medically relevant algorithm input values with the environment of the use of the medically relevant algorithm input values. If the secure channels used are of the stipulated security quality then the medically relevant algorithm input values are confidential to the three environments stipulated by the first security requirement. Second, the algorithm result values are confidential to the two environments: the environment of the source of the algorithm result values which is the service environment and the environment of the sink of the algorithm result values which is the healthcare provider local host environment. This is due to the networked service environment using a secure channel across the technical network connecting the environment of the source of the algorithm result values with the environment of the sink of the algorithm result values. If the secure channels used are of the stipulated security quality then the algorithm result values are confidential to the two environments stipulated by the second security requirement. Third, the sink of the algorithm result values which is the interface module residing within the healthcare provider local host environment can unambiguously determine the source of the medically relevant algorithm input values which is the database DB residing within the healthcare provider IT environment. This is due to the networked service environment using a secure channel across the technical network connecting the environment of the source of the medically relevant algorithm input values with the environment of the sink of the algorithm result values. If the secure channels used are of the stipulated security quality then the sink of the algorithm result values can unambiguously determine the source of the medically relevant algorithm input values. Fourth, the sink of the algorithm result values which is the interface module residing within the healthcare provider local host environment can unambiguously determine the source of the received algorithm result values which is the medical algorithm devices residing within the service environment. This is due to the networked service environment using a secure channel across the technical network connecting the environment of the sink of the algorithm result values with the environment of the source of the received algorithm result values. If the secure channels used are of the stipulated security quality, then the sink of the algorithm result values can unambiguously determine the source of the received algorithm result values.

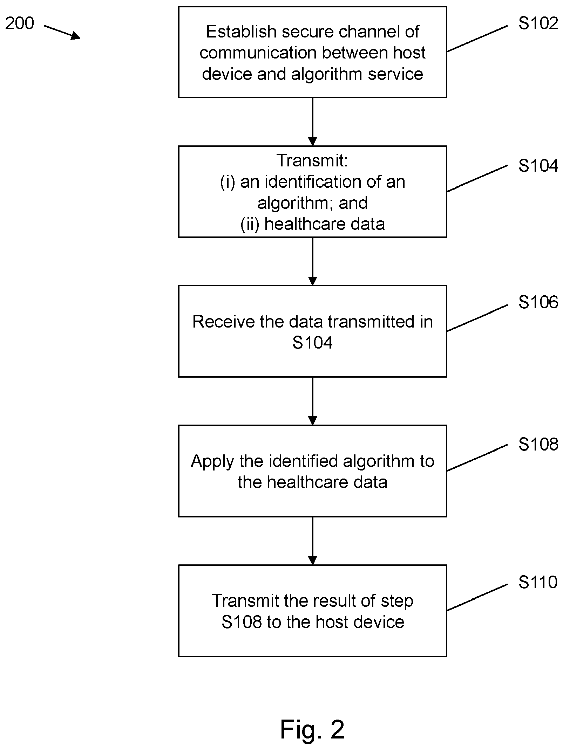

Figure 2 is a flow chart of a method 200. In a first step, S102, a secure channel of communication between a host device and an algorithm service is established. Next, in step S104, the host device transmits (i) an identification of an algorithm; and (ii) healthcare data on which the identified algorithm is to be applied to the algorithm service via the established secure channel of communication. The algorithm service then, in step S106, receives this transmitted data. The algorithm service then causes, in step S108, the identified algorithm to be applied to the healthcare data. The result of this is transmitted to the host device in step S110 via the established secure channel of communication.

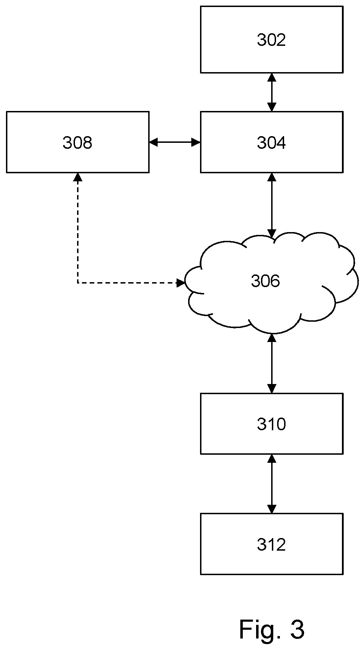

Figure 3 is a network diagram. A host device 302 of the type discussed previously is connected, for example by virtue of being on the same machine (virtual or physical) or via a network connection, to an edge module 304. The edge module 304 is connected to a wide area network (e.g. the internet) 306. In this example, the edge module 304 is also connected via a network connection to IT service 308 in a manner which does not run through wide area network 306. However in some examples, demarked by the dashed line, the IT service 308 may be connected to the wide area network and through this to the edge module 304.

An algorithm service 312 of the type discussed previously is connected, for example by virtue of being on the same machine (virtual or physical) or via a network connection, to API 310. The API 310 is connected to the wide area network 306. In this way, the API 310 and edge module 304 are able to communicate, establish the secure channel of communication, and exchange data.

The systems and methods of the above embodiments may be implemented in a computer system (in particular in computer hardware or in computer software) in addition to the structural components and user interactions described.

The term "computer system" includes the hardware, software and data storage devices for embodying a system or carrying out a method according to the above described embodiments. For example, a computer system may comprise a central processing unit (CPU), input means, output means and data storage. The computer system may have a monitor to provide a visual output display. The data storage may comprise RAM, disk drives or other computer readable media. The computer system may include a plurality of computing devices connected by a network and able to communicate with each other over that network.

The methods of the above embodiments may be provided as computer programs or as computer program products or computer readable media carrying a computer program which is arranged, when run on a computer, to perform the method(s) described above.

The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-OMs; electrical storage media such as memory, including RAM, ROM and flash memory; and hybrids and combinations of the above such as magnetic/optical storage media.

While the disclosure has been described in conjunction with the exemplary embodiments described above, many equivalent modifications and variations will be apparent to those skilled in the art when given this disclosure. Accordingly, the exemplary embodiments of the disclosure set forth above are considered to be illustrative and not limiting. Various changes to the described embodiments may be made without departing from the spirit and scope of the disclosure.

In particular, although the methods of the above embodiments have been described as being implemented on the systems of the embodiments described, the methods and systems of the present disclosure need not be implemented in conjunction with each other, but can be implemented on alternative systems or using alternative methods respectively.

The features disclosed in the description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the disclosure in diverse forms thereof.

While the disclosure has been described in conjunction with the exemplary embodiments described above, many equivalent modifications and variations will be apparent to those skilled in the art when given this disclosure. Accordingly, the exemplary embodiments of the disclosure set forth above are considered to be illustrative and not limiting. Various changes to the described embodiments may be made without departing from the spirit and scope of the disclosure.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A computer-implemented method of transferring healthcare data between a host device and an algorithm service, the host device residing within a first computer-network and the algorithm service residing within a second computer-network different to the first computer-network, the method including steps of:
(a) establishing, between the host device and the algorithm service, a secure channel of communication;
(b) transmitting, from the host device to the algorithm service via the secure channel of communication:
an identification of an algorithm to be applied to healthcare data held by the host device, and
the healthcare data to which the identified algorithm is to be applied; and
(c) receiving, at the algorithm service, the identification of the algorithm and the healthcare data;
(d) applying the identified algorithm to the healthcare data to arrive at a result; and
(e) transmitting, from the algorithm service to the host via the secure channel of communication, the result of applying the identified algorithm to the healthcare data.

2. The computer-implemented method of claim 1, wherein the method further includes transmitting, from the algorithm service to the host device, GUI instructions which, when executed by the host device, cause the generation of a graphical user interface, the GUI instructions selected by the algorithm service based on the algorithm identified by the host device.

3. The computer-implemented method of claim 1 or claim 2, the method further including establishing, between the host device and a database, a second secure channel of communication, and transmitting from the host device to the database through the second secure channel, an identification of a patient and one or more data field identifiers, the database returning a patient specific value for the or each data field identifier, wherein the patient specific value(s) are included or form the healthcare data transmitted from the host device to the algorithm service.

4. The computer-implemented method of claim 3, wherein establishing the second secure channel of communication includes one or both of:
the host device authenticating the identity of the database; and/or
the host device and the database each encrypting messages to be sent between each other.

5. The computer-implemented method of any preceding claim, the method further including a step, between steps c) and (d), of transmitting, from the algorithm service to the host device, confirmatory instructions indicating which algorithm is to be applied to what healthcare data.

6. The computer-implemented method of claim 5, wherein the confirmatory instructions comprise confirmatory GUI instructions which cause the generation of a pre-populated graphical user interface illustrating the healthcare data provided and the algorithm to be applied.

7. The computer-implemented method of claim 6, wherein the method further comprises receiving at the host device one or more change requests relating to the pre-populated graphical user interface, and transmitting these change requests to the algorithm service.

8. The computer-implemented method of any preceding claim, further including a step of transforming the result of applying the algorithm to the healthcare data before transmitting it to the host device and/or a step of transforming the healthcare data before transmitting it to the algorithm service.

9. The computer-implemented method of any preceding claim, the algorithm service including an API and the host device including an edge module, the API and edge module being in secure communication via the secure channel of communication.

10. The computer-implemented method of any preceding claim, wherein establishing the secure channel of communication includes the host device authenticating the identity of the algorithm service.

11. The computer-implemented method of any preceding claim, wherein establishing the secure channel of communication includes the host device and algorithm service each encrypting messages to be sent between each other.

12. A host device, comprising one or more processors, memory, and a network interface, the memory containing machine executable instructions which, when executed on the processor(s) cause:
(a) the network interface to establish a secure channel of communication between the host device and an algorithm service, the host device residing within a first computer-network and the algorithm service residing within a second computer-network different to the first computer-network;
(b) the host device to transmit, to the algorithm service via the secure channel of communication:
an identification of an algorithm to be applied to healthcare data held by the host device, and
the healthcare data to which the identified algorithm is to be applied; and
(c) the host device to receive, from the algorithm service via the secure channel of communication, the result of applying the identified algorithm to the healthcare data.

13. An algorithm service, comprising one or more processors, memory, and a network interface, the memory containing machine executable instructions which, when executed on the processor(s) cause:
(a) the network interface to establish a secure channel of communication between the algorithm service and a host device, the host device residing within a first computer-network and the algorithm service residing within a second computer-network different to the first computer-network;
(b) the algorithm service to receive, from the host device via the secure channel of communication:
an identification of an algorithm to be applied to healthcare data held by the host device, and
the healthcare data to which the algorithm is to be applied;
(c) the algorithm service to apply the identified algorithm to the healthcare data to arrive at a result; and
(e) the algorithm service to transmit, to the host device via the secure channel of communication, the result of applying the identified algorithm to the healthcare data.

14. An edge module for a host device, configured to:
(a) establish a secure channel of communication between the edge module and an algorithm service, the edge module residing within a first computer-network and the algorithm service residing within a second computer-network different to the first computer-network;
(b) receive, from an interface module, an identification of an algorithm to be applied to healthcare data held by the host device or received from the interface module;
(c) transmit, to the algorithm service via the secure channel of communication:
an identification of the algorithm to be applied to the healthcare data held by the host device, and
the healthcare data to which the identified algorithm is to be applied; and
(d) receive, from the algorithm service via the secure channel of communication, the result of applying the identified algorithm to the healthcare data.

15. A system comprising:
a host device residing within a first computer-network, and
an algorithm service residing within a second computer-network different to the first computer network;
the host device and/or the algorithm service being configured to:
establish a secure channel of communication between the host device and the algorithm service;
the host device being configured to:
transmit, to the algorithm service via the secure channel of communication:
an identification of an algorithm to be applied to healthcare data held by the host device, and
the healthcare data to which the identified algorithm is to be applied;
the algorithm service being configured to
apply the identified algorithm to the healthcare data to arrive at a result; and
transmit, to the host device via the secure channel of communication, the result of applying the identified algorithm to the healthcare data.
